# EUROPEAN PATENT SPECIFICATION

(11) **EP 4 601 494 B1**
(45) Date of publication and mention of the grant of the patent: **25.11.2026**
(21) Application number: 23821734.3
(22) Date of filing: 10.10.2023
(51) Int. Cl.: A41D 13/06, A41D 13/08, A61F 13/06

(54) **PROTECTION DEVICE FOR FINGERS AND TOES**
FINGER- UND ZEHENSCHUTZVORRICHTUNG
DISPOSITIF DE PROTECTION POUR DOIGTS ET ORTEILS

(30) Priority: 10.10.2022 IT 202200020799
(43) Date of publication of application: 20.08.2025
(73) Proprietor: Lupo, Sandro, 90143 Palermo (IT)
(72) Inventor: Lupo, Sandro, 90143 Palermo (IT)
(74) Representative: Dalla Rosa, Silvia
(86) International application number: PCT/IB2023/060182
(87) International publication number: WO 2024/079635

(56) References cited:
- ES-U- 1 035 012
- ES-U- 1 254 589
- US-A1- 2021 030 084

## Description

### Technical Field

The present invention find application into the technical field of devices and preparations for medical and cosmetic use and particularly has as its object a protective device for finger and toes, to be used in particular with or for the prevention of calluses.

### State of the art

Special plasters are known for the treatment or prevention of calluses, commonly known as paracallus, which usually comprise a layer of elastomeric material having a greater thickness than common plasters for the treatment of skin wounds and which has a central hole which will be positioned in correspondence of the callus.

In this way, the plaster will prevent rubbing of the part affected by the callus, for example due to the presence of the sock or shoe, alleviating the discomfort that would result.

A first type of callus protector, which is particularly widespread on the market, requires the plaster to be made up of a simple circular element with a hole in the middle and having an adhesive side to be attached to the skin.

This solution, however, is limited by poor stability as the fixing is entrusted exclusively to the adhesive layer, with the consequence that the plaster tends to easily move, losing its effectiveness.

Another type involves the addition of fixing fins, similar to common wound plasters, so as to increase stability.

Even these solutions, however, have proven to be inefficient, above all due to the fact that the thicknesses of common callus protectors are reduced and not adaptable to the shape of the callus.

Not least, the well-known callus protectors are poorly adaptable to the anatomy of the specific user, so that they can be uncomfortable to wear.

Other solutions involve the use of simple elastomeric sleeves to be put on the finger on which the callus is present.

These solutions, by not leaving the callus free, do not promote its natural disappearance and do not solve the problem of rubbing.

Last but not least, they are adaptable to the configuration of the finger in a limited way and dependent on the elasticity of the material.

Document US 2021/030084 discloses a protection device according to the preamble of claim 1.

### Scope of the invention

The object of the present invention is to overcome the above-mentioned drawbacks, providing a device for the protection of finger and toes affected by calluses which is characterized by high efficiency and relative cost-effectiveness.

A particular object is to provide a device for protecting fingers or toes whose configuration may be adapted to the anatomical configuration of the finger or toe to which it is to be applied.

Yet another object is to provide a device for protecting the fingers or toes whose configuration may be adapted to the specific position of the callus on the finger or toe to be treated.

Yet another object is to provide a device for the protection of fingers and toes which is thicker than traditional callus protector and which is also adjustable according to needs.

These objects, as well as others which will become more apparent hereinafter, are achieved by a protection device for fingers and toes which, according to claim 1, comprises a tubular element suitable for being fitted on the toe of a foot or a finger of a hand at a callus, a projection extending from the side wall of said tubular element, a through hole made in said projection and in said tubular element to be placed on the callus, wherein said tubular element and/or said projection have one or more weakening or pre-cut lines for the removal of respective portions to allow the modification of their respective forms.

In this way it will be possible to modify the configuration of the device acting as a callus protector based on the length of the finger or toe to be treated and/or the thickness of the callus and/or the specific position of the callus along the finger or toe. In particular, the protective device, once fitted on the affected finger or toe, will counteract the friction that is generated between the fingers or toes, especially between the toes, in correspondence with the middle and distal phalanges at the height of the joint capsules, and with the footwear.

The device will isolate and protect these areas to ensure comfort during walking. Advantageous embodiments of the invention are obtained in accordance with the dependent claims.

### Brief disclosure of the drawings

Further features and advantages of the invention will be become more apparent in light of the detailed description of preferred but not exclusive embodiments of the protection device, illustrated by way of non-limiting example with the aid of the attached drawing tables wherein:
**FIG. 1** is a perspective view of the device in the complete configuration according to a first embodiment;
**FIG. 2** is a top view of the device of Fig. 1;
**FIG. 3** is a perspective view of the device in the first embodiment and in a second operative configuration;
**FIG. 4** is a perspective view of the device in the first embodiment and in a third configuration;
**FIG. 5** is a side view of the device of Fig. 4;
**FIG. 6** is a perspective view of the device in the first embodiment and in a fourth configuration;
**FIG. 7** is a side view of the device of Fig. 6;
**FIG. 8** is a perspective view of the device in the first embodiment and in a fifth configuration;
**FIG. 9** is a perspective view of the device in the first embodiment and in a sixth configuration;
**FIGs. 10****,** **13****,** **15****,** **17****,** **19** are perspective views of the device in a second embodiment and according to different operative configurations obtained by removing various portions;
**FIGs. 11****,** **14****,** **16****,** **18****,** **20** are side views of the device respectively according to Figs. 10, 13, 15, 17, 19;
**FIG. 12** is a sectional side view of the device of Fig. 11.

### Best modes of carrying out the invention

With reference to the attached figures, some possible configurations are illustrated, exemplifying and not limiting, of a finger or toe protection device according to the present invention.

The protective device will be intended to be worn on one of the toes or fingers in order to treat a callus or prevent its formation.

The protection device, indicated globally with **1,** first of all comprises a tubular element **2** having a side wall **3** with a central development axis **L** which defines the longitudinal direction of development of the tubular element **2.**

The tubular element **2** is suitable for being fitted on the toe or finger in correspondence with a callus or an area where its formation is to be prevented.

Furthermore, the tubular element **2** is integral with a projection **4** which extends radially from the side wall **3** of the tubular element **2** and is provided with a through hole **5** which also passes through the tubular element **2** itself to be placed on the callus or on the part of the finger or toe to be treated, even in preventive mode.

In particular, the through hole has a radial central axis **X** which may be inclined or more preferably orthogonal with respect to the longitudinal axis **L** of the side wall **3** of the tubular element **2.**

The tubular element **2** and/or the projection **4** have one or more weakening or pre-cut lines (**10,11**) for the removal of respective portions to allow the modification of their respective forms or shapes and thus obtain one or more different configurations of use in order to preserve multiple areas simultaneously and approach more effectively the stresses in the relaxation phase when the fingers and toes relax and expand their support surface.

To use the protective device **1** and wear it on one of the toes or finger of a foot or hand, it will be necessary to guide the perforated tubular element **2** in correspondence with the bony prominence of the phalanx so that its position creates a distance between the toes or from the shoe and at the same time isolate the sensitive area.

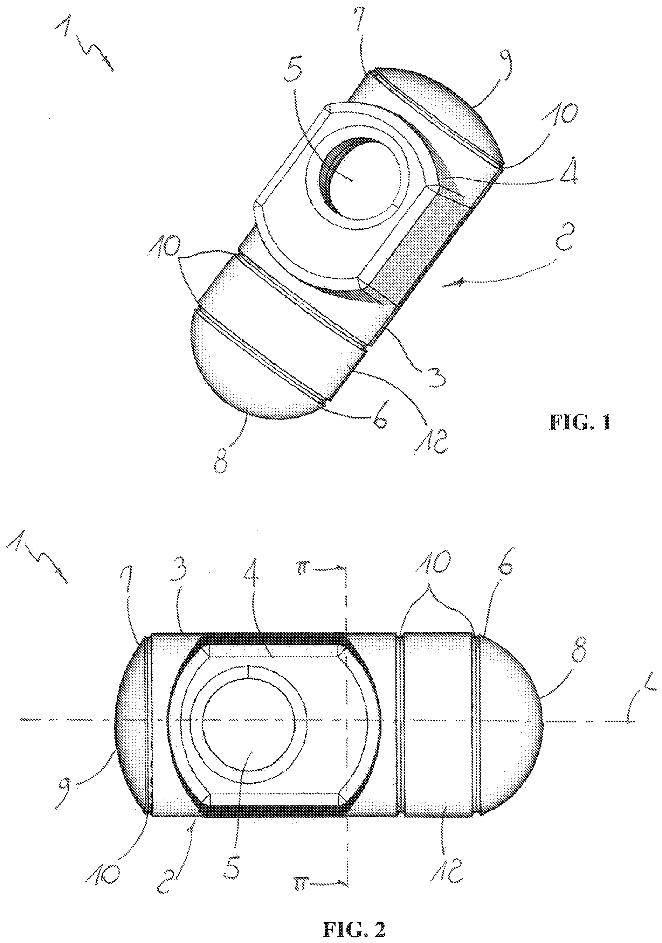

**Fig. 1** shows a possible starting configuration of the protection device **1** wherein the tubular element **2** is provided with end portions **6, 7** closed by respective front caps **8, 9** suitable to be removed from the tubular element **2** according to the intended use.

From **Fig. 2****,** however, it can be observed that the position of the projection **4** is not centered with respect to a radial and transversal center plane **π** of the tubular element **2,** so that the central axis **X** of the hole **5** is offset along a longitudinal direction with respect to the center plane **π.**

In this way, the flexibility of use of the device **1** will be increased since, based on which of the two caps **8, 9** will be removed, a configuration of different length will be obtained which can be more easily adapted to the size of the finger or toe to be treated and/or the position of the callus along the finger or toe and/or the position of the area to be treated along the finger or toe.

According to the invention, the tubular element **2** is provided with one or more weakening or pre-cut lines **10** designed to facilitate the removal of one or more portions and obtain respective configurations of the protection device **1.**

Therefore, one or both of the front caps **8, 9** are removable by removing one or both of the respective end portions in correspondence with a respective weakening or pre-cut line **10.**

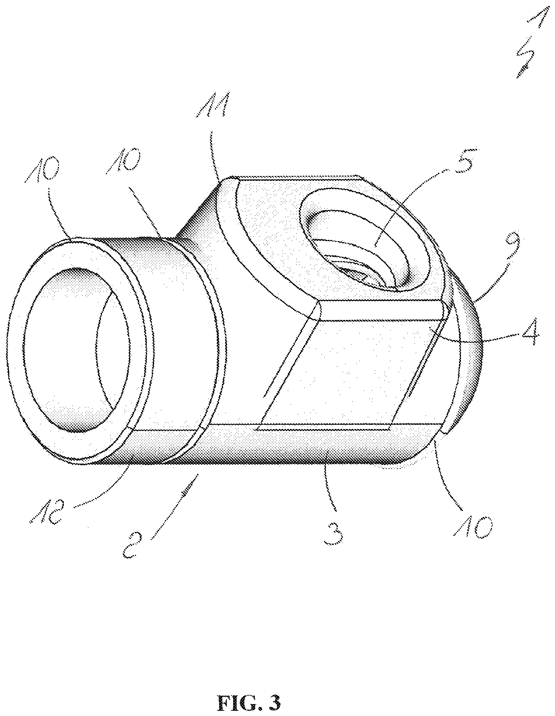

For example, **Fig. 3** shows a configuration wherein one of the two end caps has been removed, in particular the cap **8** at the furthest end along the longitudinal axis **L** of the tubular element **2** with respect to the central axis **X** of hole **5.**

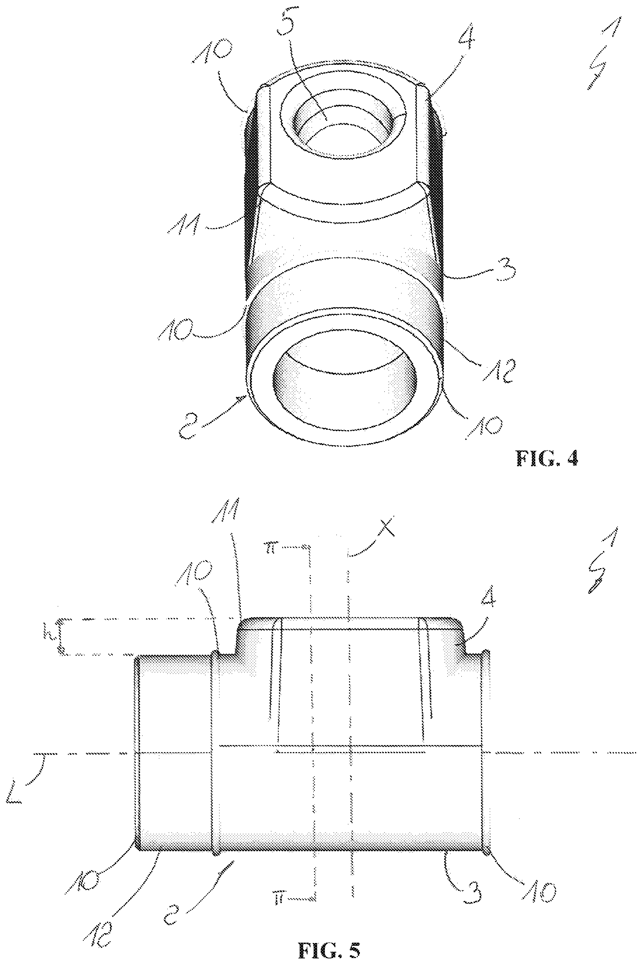

**Fig. 5** shows a further configuration wherein both end caps **8, 9** have been removed by removing the respective end portions **6, 7** in correspondence with the appropriate weakening or pre-cut lines **10.**

This configuration will allow the protection device **1** to be fitted also at the intermediate phalanges.

According to these first embodiments, the weakening lines **10** made on the tubular element **2** will be circular and will extend perimeterally along the entire side wall **3** of the tubular element **2,** coaxially thereto, so as to remove portions which are also tubular and so that the sections of the tubular element **2** at which there will be the open openings will have a substantially circular shape.

The configurations of **Figs.** from **10** to **20** show a second embodiment wherein the weakening or pre-cut lines **10** are placed on respective cutting planes inclined and not orthogonal to the longitudinal axis **L** of the tubular element **2,** so that the sections of the tubular element **2** at which there will be the openings will have a substantially elliptical shape.

Advantageously, regardless of the configuration of the weakening or pre-cut lines **10,** the projection **4** may also be provided with one or more weakening or pre-cut lines **11** suitable for allowing the removal of one or more portions and allowing the adjustment of the height **h.**

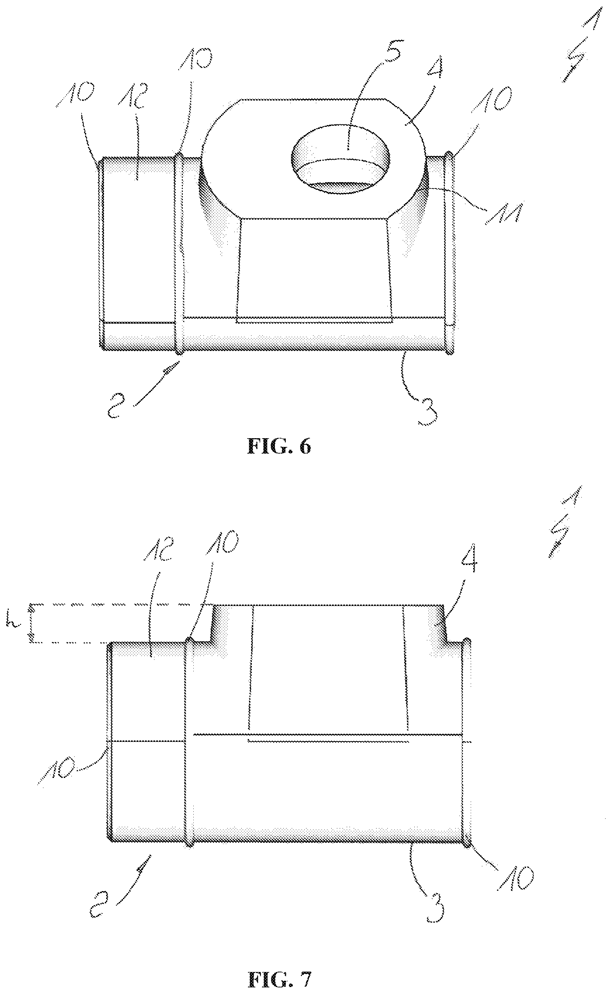

Furthermore, preferably, the projection **4** will have a square shape in plan with beveled corners to better accommodate the bony prominences of the phalanges (joint capsules). For example, **Figs. 6** and **7****,** relating to the first embodiment, illustrate respective configurations in which the tubular element **2** has been reduced in length by removing both the two end portions **6, 7** provided with caps **8, 9** and in which the height **h** of the projection **4** has been reduced.

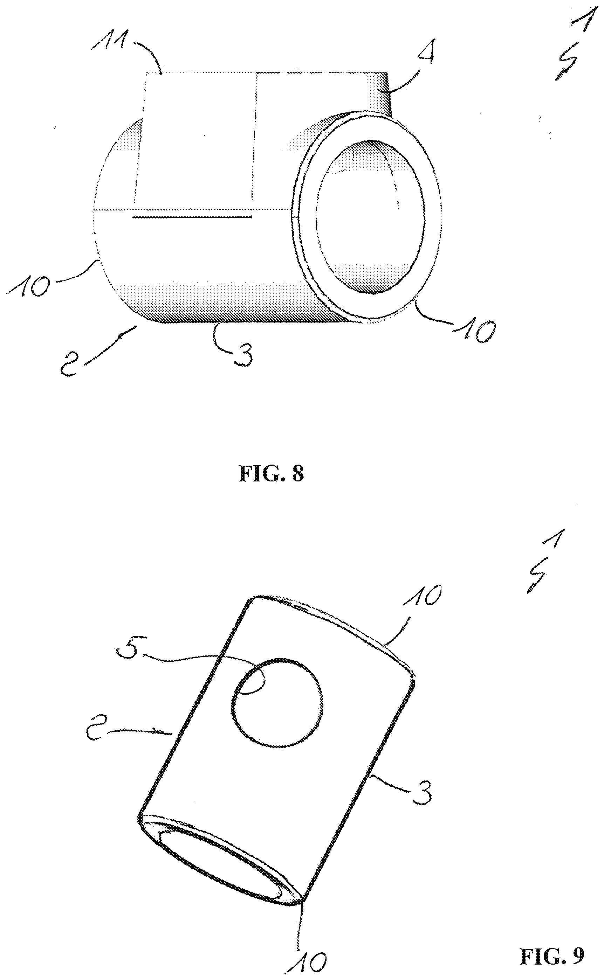

**Fig. 8** shows a further variant wherein the tubular element **2** has been further shortened longitudinally by removing a further longitudinal tubular portion **12.**

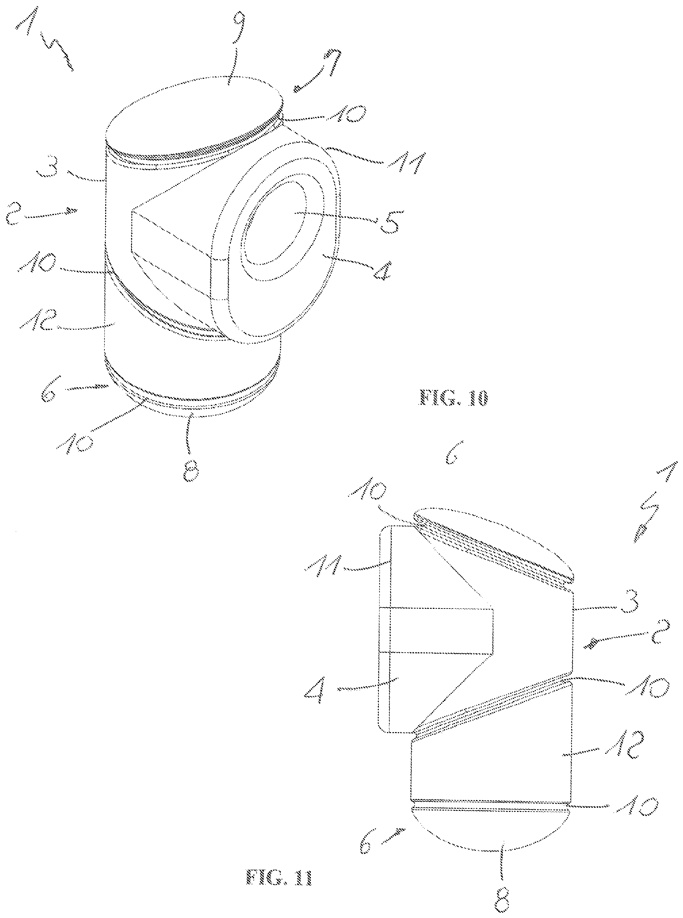

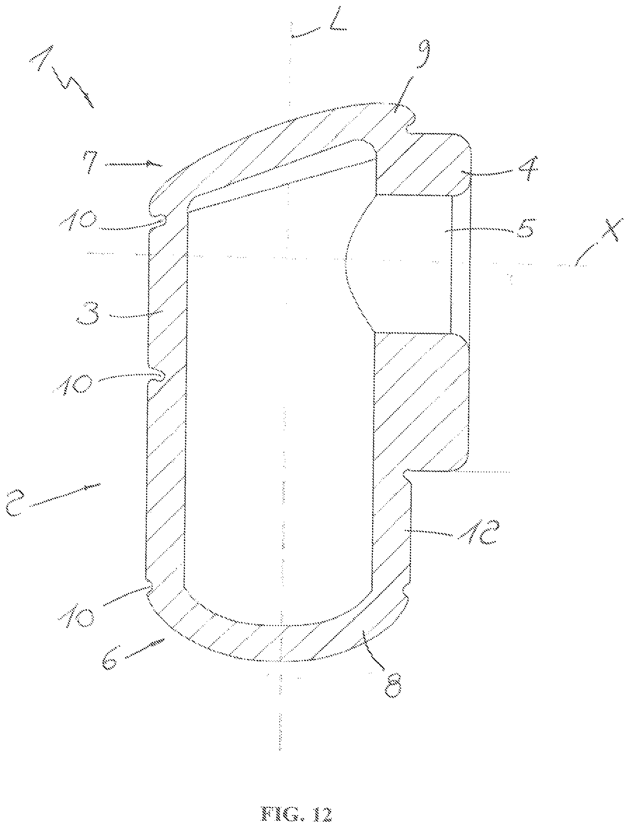

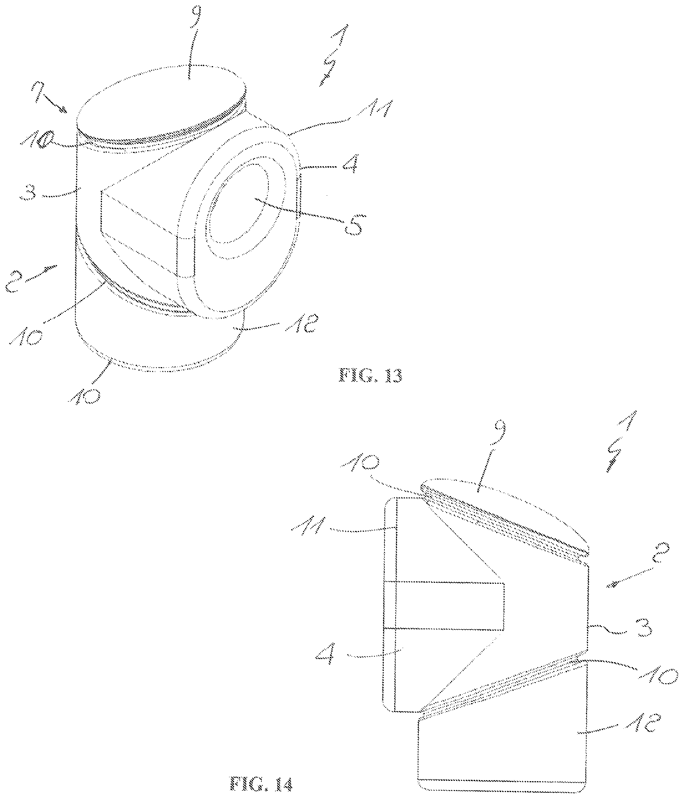

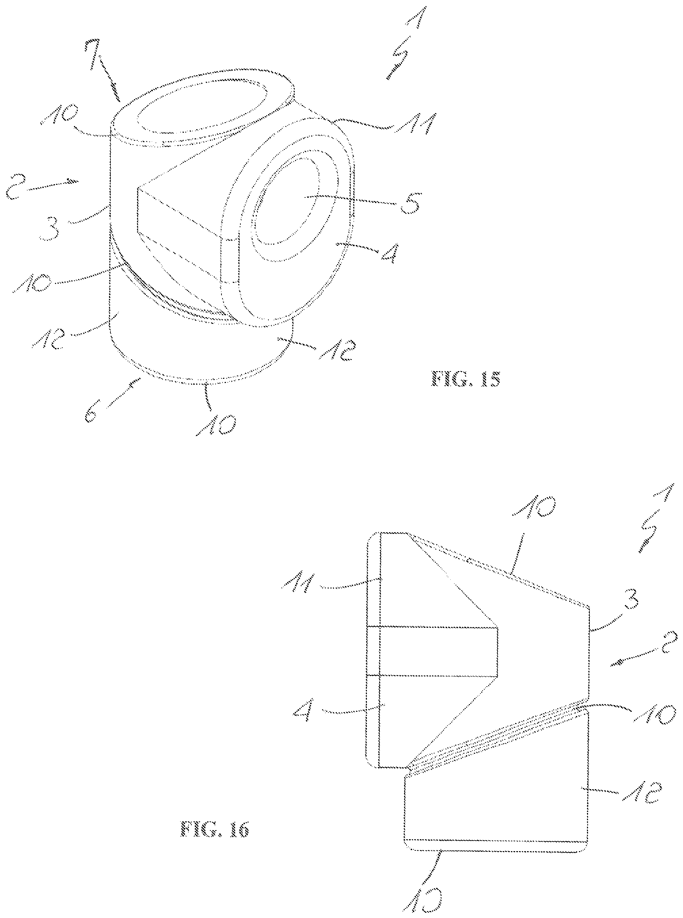

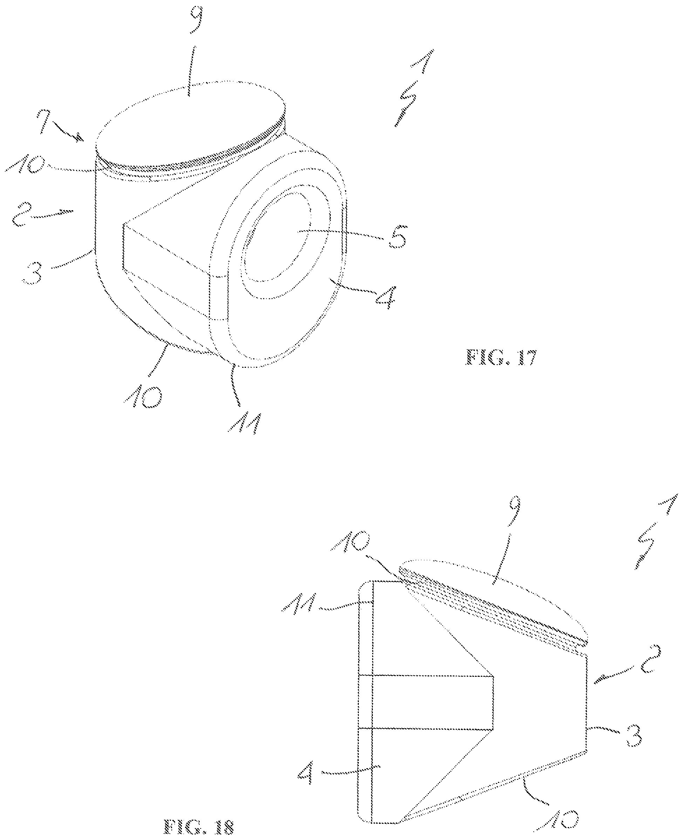

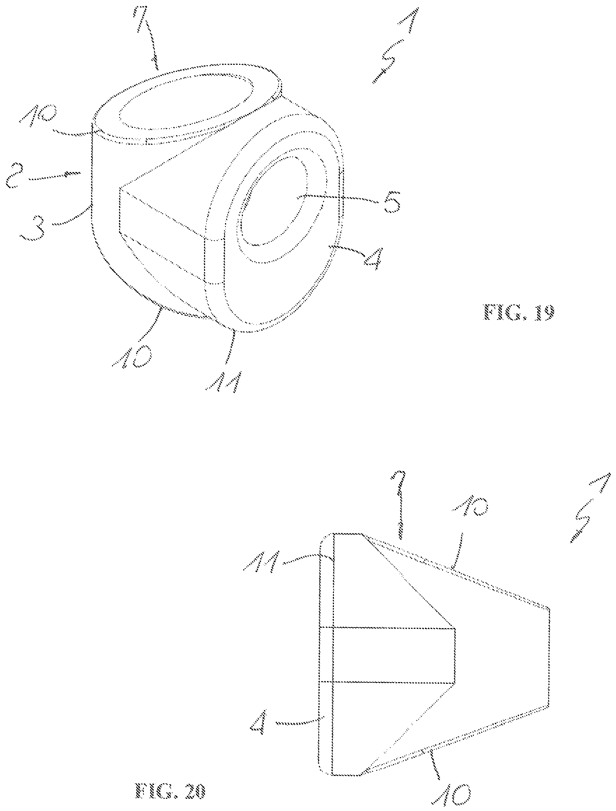

**Fig. 9** shows a further variant wherein the projection **4** has been completely removed. Similarly, **Figs. 10** to **20** show the various configurations that can be obtained starting from a device in its complete configuration, illustrated in **Fig. 10****,** by removing one or both caps **8, 9** and possibly the longitudinal tubular portion **12.**

It is understood that it will be possible to obtain further configurations different from those illustrated based on which portions of the tubular element **2** and the projection **4** will be removed, also providing a different distribution and/or number of weakening or pre-cut lines **10, 11.**

The possibility of modifying the height **h** of the projection **4** or eliminating it completely will allow a further possibility of adapting the protection device **1** to the specific needs as well as to the dimensions of any callus to be treated.

Furthermore, by making a longitudinal cut, even in the absence of pre-cut lines, at the section immediately behind the projection **4,** it will be possible to separate and isolate the projection **4** from the tubular element **2** and use the former as a measuring device suitable to allow the user or even a podiatrist to determine the size of the most suitable protective device for the specific user.

Conveniently, the entire protective device **1** will be made of silicone, preferably of medical grade and in the form of a polymeric gel.

## Claims

1. A protection device for fingers and toes, comprising:
- a tubular element **(2)** having a side wall **(3)** defining a central axis of development **(A)** and suitable to be fitted on the toe or finger at a callus or at an area to be treated;
**characterized by:**
- a projection **(4)** which extends from said side wall **(3)** of said tubular element **(2)**;
- a through hole **(5)** made in said projection **(4)** and in said tubular element **(2)** to be placed on the callus or on the area to be treated;
wherein said tubular element **(2)** and/or said projection **(4)** have one or more weakening or pre-cut lines **(10, 11)** for the removal of respective portions to allow the modification of their respective forms.

2. Protection device as claimed in claim 1, **characterized in that** said tubular element **(2)** is closed at one or both ends by front caps **(8, 9)** suitable to be selectively removed from said tubular element **(2)**.

3. Protection device as claimed in any preceding claim, **characterized in that** said front caps **(8, 9)** are removable by removing a respective end portion **(6, 7)** at one of said weakening lines **(10)**.

4. Protection device as claimed in any preceding claim, **characterized in that** one or more of said weakening lines **(10)** are made on said tubular element **(2)** and extend perimetrically to the side wall **(3)** thereof.

5. Protection device as claimed in claim 4, **characterized in that** one or more of said weakening lines **(10)** extend perimetrically to the side wall **(3)** of said tubular element (2) for the entire perimeter development of said side wall **(3)**.

6. Protection device as claimed in claim 4 or 5, **characterized in that** one or more of said weakening lines **(10)** are circular and extend perimetrically to said side wall **(3)** of said tubular element **(2)**, coaxially thereto.

7. Protection device as claimed in any claim 4 to 6, **characterized in that** one or more of said weakening lines **(10)** develop along respective planes inclinated with respect to the central axis of said side wall **(3)** of said tubular element **(2)**.

8. Protection device as claimed in any preceding claim, **characterized in that** said projection **(4)** has a height **(h)** that can be adjusted by removing a respective portion at a corresponding one of said weakening lines **(11)**.

9. Protection device as claimed in any preceding claim, **characterized in that** said through hole **(5)** has a central axis **(X)** orthogonal to said side wall **(3)** of said tubular element **(2)**.

10. Protection device as claimed in claim 9, **characterized in that** said central axis **(X)** of said through hole **(5)** is offset with respect to a radial center plane **(π)** of said tubular element **(2)**.

11. Protection device as claimed in any preceding claim, **characterized in that** said projection **(4)** has a square shape in plan with rounded corners.

12. Protection device as claimed in any preceding claim, **characterized in that** it is made of medical silicone.

## Patentansprüche

1. Schutzvorrichtung für Finger und Zehen, umfassend:
- ein rohrförmiges Element (**2**) mit einer Seitenwand (**3**), die eine zentrale Erstreckungsachse (A) definiert, und das dazu geeignet ist, im Bereich einer Schwiele oder eines zu behandelnden Bereichs über eine Zehe oder einen Finger gezogen zu werden;
**gekennzeichnet durch**:
- einen Vorsprung (**4**), der sich von der Seitenwand (**3**) des rohrförmigen Elements (**2**) erstreckt;
- ein Durchgangsloch (**5**), das in dem Vorsprung (**4**) und in dem rohrförmigen Element (**2**) ausgebildet ist, um auf der Schwiele oder auf dem zu behandelnden Bereich platziert zu werden;
wobei das rohrförmige Element (**2**) und/oder der Vorsprung (**4**) eine oder mehrere Schwächungs- oder Vorschnittlinien **(10, 11)** zum Entfernen jeweiliger Abschnitte aufweisen, um die Änderung ihrer jeweiligen Formen zu ermöglichen.

2. Schutzvorrichtung nach Anspruch 1, **dadurch gekennzeichnet, dass** das rohrförmige Element (**2**) an einem oder beiden Enden durch Stirnkappen (**8, 9**) verschlossen ist, die selektiv von dem rohrförmigen Element (**2**) entfernt werden können.

3. Schutzvorrichtung nach einem der vorhergehenden Ansprüche, **dadurch gekennzeichnet, dass** die Stirnkappen (**8, 9**) durch Entfernen eines jeweiligen Endabschnitts **(6, 7)** an einer der Schwächungslinien **(10)** entfernbar sind.

4. Schutzvorrichtung nach einem der vorhergehenden Ansprüche, **dadurch gekennzeichnet, dass** die eine oder mehreren der Schwächungslinien **(10)** an dem rohrförmigen Element (**2**) ausgebildet sind und sich in Umfangsrichtung entlang dessen Seitenwand (**3**) erstrecken.

5. Schutzvorrichtung nach Anspruch 4, **dadurch gekennzeichnet, dass** eine oder mehrere der Schwächungslinien **(10)** sich in Umfangsrichtung der Seitenwand **(3)** des rohrförmigen Elements (2) über die gesamte Umfangserstreckung der Seitenwand **(3)** erstrecken.

6. Schutzvorrichtung nach Anspruch 4 oder 5, **dadurch gekennzeichnet, dass** eine oder mehrere der Schwächungslinien **(10)** kreisförmig sind und sich in Umfangsrichtung der Seitenwand **(3)** des rohrförmigen Elements **(2)**, koaxial dazu erstrecken.

7. Schutzvorrichtung nach einem der Ansprüche 4 bis 6, **dadurch gekennzeichnet, dass** eine oder mehrere der Schwächungslinien **(10)** entlang jeweiliger Ebenen verlaufen, die bezüglich der zentralen Achse der Seitenwand **(3)** des rohrförmigen Elements **(2)** geneigt sind.

8. Schutzvorrichtung nach einem der vorhergehenden Ansprüche, **dadurch gekennzeichnet, dass** der Vorsprung **(4)** eine Höhe **(h)** aufweist, die durch Entfernen eines jeweiligen Abschnitts an einer entsprechenden der Schwächungslinien **(11)** einstellbar ist.

9. Schutzvorrichtung nach einem der vorhergehenden Ansprüche, **dadurch gekennzeichnet, dass** das Durchgangsloch **(5)** eine zentrale Achse **(X)** aufweist, die orthogonal zu der Seitenwand **(3)** des rohrförmigen Elements **(2)** verläuft.

10. Schutzvorrichtung nach Anspruch 9, **dadurch gekennzeichnet, dass** die zentrale Achse **(X)** des Durchgangslochs **(5)** bezüglich einer radialen Mittelebene **(n)** des rohrförmigen Elements **(2)** versetzt ist.

11. Schutzvorrichtung nach einem der vorhergehenden Ansprüche, **dadurch gekennzeichnet, dass** der Vorsprung **(4)** in der Draufsicht eine quadratische Form mit abgerundeten Ecken aufweist.

12. Schutzvorrichtung nach einem der vorhergehenden Ansprüche, **dadurch gekennzeichnet, dass** sie aus medizinischem Silikon hergestellt ist.

## Revendications

1. Dispositif de protection pour les doigts et les orteils, comprenant :
- un élément tubulaire (**2**) ayant une paroi latérale (**3**) définissant un axe central de développement (A) et apte à être enfilé sur un orteil ou un doigt au niveau d'un durillon ou d'une zone à traiter ;
**caractérisé par** :
- une saillie (**4**) qui s'étend à partir de ladite paroi latérale (**3**) dudit élément tubulaire (**2**) ;
- un trou traversant **(5)** pratiqué dans ladite saillie (**4**) et dans ledit élément tubulaire (**2**) pour être placé sur le durillon ou sur la zone à traiter ;
dans lequel ledit élément tubulaire (**2**) et/ou ladite saillie (**4**) présentent une ou plusieurs lignes d'affaiblissement ou de pré-découpe **(10, 11)** pour l'enlèvement de portions respectives afin de permettre la modification de leurs formes respectives.

2. Dispositif de protection selon la revendication 1, **caractérisé en ce que** ledit élément tubulaire (**2**) est fermé à une ou aux deux extrémités par des capuchons frontaux (**8, 9**) qui peuvent être sélectivement retirés dudit élément tubulaire (**2**).

3. Dispositif de protection selon l'une quelconque des revendications précédentes, **caractérisé en ce que** lesdits capuchons frontaux (**8, 9**) sont amovibles par enlèvement d'une portion d'extrémité respective (**6, 7**) au niveau de l'une desdites lignes d'affaiblissement (**10**).

4. Dispositif de protection selon l'une quelconque des revendications précédentes, **caractérisé en ce qu'**une ou plusieurs desdites lignes d'affaiblissement (**10**) sont réalisées sur ledit élément tubulaire (**2**) et s'étendent de manière périmétrique par rapport à la paroi latérale (**3**) de celui-ci.

5. Dispositif de protection selon la revendication 4, **caractérisé en ce qu'**une ou plusieurs desdites lignes d'affaiblissement **(10)** s'étendent de manière périmétrique par rapport à la paroi latérale **(3)** dudit élément tubulaire (2) sur tout le développement périmétrique de ladite paroi latérale **(3).**

6. Dispositif de protection selon la revendication 4 ou 5, **caractérisé en ce qu'**une ou plusieurs desdites lignes d'affaiblissement **(10)** sont circulaires et s'étendent de manière périmétrique par rapport à ladite paroi latérale **(3)** dudit élément tubulaire **(2)**, coaxialement à celle-ci.

7. Dispositif de protection selon l'une quelconque des revendications 4 à 6, **caractérisé en ce qu'**une ou plusieurs desdites lignes d'affaiblissement **(10)** se développent le long de plans respectifs inclinés par rapport à l'axe central de ladite paroi latérale **(3)** dudit élément tubulaire **(2)**.

8. Dispositif de protection selon l'une quelconque des revendications précédentes, **caractérisé en ce que** ladite saillie **(4)** a une hauteur **(h)** pouvant être réglée par enlèvement d'une portion respective au niveau de l'une correspondante desdites lignes d'affaiblissement **(11)**.

9. Dispositif de protection selon l'une quelconque des revendications précédentes, **caractérisé en ce que** ledit trou traversant **(5)** a un axe central **(X)** orthogonal à ladite paroi latérale **(3)** dudit élément tubulaire **(2)**.

10. Dispositif de protection selon la revendication 9, **caractérisé en ce que** ledit axe central **(X)** dudit trou traversant **(5)** est décalé par rapport à un plan radial central **(n)** dudit élément tubulaire **(2)**.

11. Dispositif de protection selon l'une quelconque des revendications précédentes, **caractérisé en ce que** ladite saillie **(4)** a une forme carrée en plan avec des coins arrondis.

12. Dispositif de protection selon l'une quelconque des revendications précédentes, **caractérisé en ce qu'**il est réalisé en silicone médical.
